**Europäisches Patentamt**

**European Patent Office**

**Office européen des brevets**

(19)

(11) Veröffentlichungsnummer: **0 012 153**

**B2**

(12) # NEUE EUROPÄISCHE PATENTSCHRIFT

(45) Veröffentlichungstag der neuen Patentschrift:
02.07.86

(21) Anmeldenummer: 79102931.7

(22) Anmeldetag: 13.08.79

(51) Int. Cl.⁴: **B 01 J  23/58,** C 07 C  85/11,
B 01 J  23/54, B 01 J  37/02

(54) **Herstellung und Verwendung von Trägerkatalysatoren.**

(30) Priorität: 11.11.78  DE 2848978

(43) Veröffentlichungstag der Anmeldung:
25.06.80 Patentblatt 80/13

(45) Bekanntmachung des Hinweises auf die Patenterteilung:
31.03.82 Patentblatt 82/13

(45) Bekanntmachung des Hinweises auf die Entscheidung
über den Einspruch:
02.07.86 Patentblatt 86/27

(84) Benannte Vertragsstaaten:
**AT BE DE FR GB IT NL SE**

(56) Entgegenhaltungen:
**DE-A-1 944 933
DE-A-2 135 155
DE-A-2 448 449
FR-A-2 093 725
GB-A-1 283 737**

**J.R. Anderson: Structure of Metallic Catalysts
London (Academic Press) 1975**

**Die Akte enthält technische Angaben, die nach
dem Eingang der Anmeldung eingereicht wurden
und die nicht in dieser Patentschrift enthalten sind.**

(73) Patentinhaber: **BAYER AG, Konzernverwaltung RP
Patentabteilung, D-5090 Leverkusen 1 Bayerwerk
(DE)**

(72) Erfinder: **Birkenstock, Udo, Dr., Schneppersdelle
2, D-4030 Ratingen 8 (DE)**
Erfinder: **Schmidt, Herbert,, Im Hederichsfeld 52,
D-5090 Leverkusen 3 (DE)**

EP 0 012 153 B2

LIBER, STOCKHOLM 1986

## Beschreibung

Die vorliegende Erfindung betrifft neuartige, hochaktive Trägerkatalysatoren und ein Verfahren zu ihrer Herstellung durch Vorbehandlung von inerten Trägermaterialien mit Basen und Trocknung derselben bis zur Gewichtskonstanz vor der Aufbringung der katalytisch wirksamen Komponente, sowie die Verwendung der Trägerkatalysatoren.

Unter Trägerkatalysatoren werden Zusammensetzungen verstanden, die ein oder mehrere vorgeformten Trägermaterialien enthalten und für katalytische Zwecke eingesetzt werden. Derartige Katalysatoren sind unter den verschiedensten Bezeichnungen gebräuchlich und handelsüblich, wobei die Wirkstoffe, insbesondere die Edelmetalle, in feinstverteilter Form entweder als Metall oder als Salz auf Trägern wie Siliciumdioxid, Aluminiumoxid, Magnesium- und Aluminiumsilikaten, Carbonaten u. a. aufgebracht sind. Einige konkrete Beispiele für Trägerkatalysatoren sind Pd/$\alpha$-Al$_2$O$_3$ Strangpreßlinge, Pd/V$_2$O$_5$/LiAl-Spinell-Kugeln, Platin, Palladium, Rhodium, Ruthenium oder Silber auf Aluminiumsilikat. Ganz besondere Bedeutung haben diejenigen Katalysatoren erlangt, die Edelmetalle auf Trägern mit einer niedrigen BET-Oberfläche, z. B. <50m$^2$/g enthalten. Sie werden durch Tränken des entsprechenden Trägermaterials mit einer wäßrigen Lösung von Metall- und/oder Edelmetallsalzen und durch anschließende Reduktion erhalten. Die so hergestellten Katalysatoren enthalten eine unregelmäßige Wirkstoffverteilung über den Träger. Sie finden Verwendung bei den verschiedensten katalytischen Prozessen, z. B. bei der Oxidation, Polymerisation, Hydrierung, Dehydrierung, Epoxidierung, Vinylierung oder beim hydrierenden Cracken. Bei der Herstellung solcher Katalysatoren sind eine Reihe von Maßnahmen erforderlich, die für den späteren technischen Einsatz der Katalysatoren entscheidend sein können, z. B. wenn es gilt, die Bildung unerwünschter Nebenprodukte zu verhindern. Beispielsweise muß der Einfluß der Oberfläche des Trägermaterials als Ursache für die Nebenproduktbildung berücksichtigt werden. Ferner kan es vorkommen, daß die im Inneren eines Trägerkorns, d. h. bis zum Zentrum, vorhandenen Wirksubstanzen entweder gar nicht oder nur in untergeordnetem Maße an der zu katalysierenden Reaktion teilnehmen.

Für viele katalytische Prozesse, wie z. B. Hydrierungen, werden Al-Spinelle, insbesondere Alkali- oder Erdalkali-Aluminiumspinelle, benutzt. Bei der Herstellung solcher Alkali- oder Erdalkali-Aluminiumspinelle bleiben verfahrensbedingt unterschiedliche Restmengen wasserlöslichen Alkalis bzw. Erdalkalis im Trägerkorn verteilt. Durch diese Inhomogenität des Spinells ergibt sich zwangsläufig eine unterschiedliche Abscheidung der Wirksubstanz bei den einzelnen Trägerkörnern. Als Folge davon können beim technischen Einsatz dieser Aluminiumspinell-Katalysatoren unerwünschte Aktivitäts-, Selektivitäts- und Standzeit-Unterschiede auftreten.

Die im Inneren eines Trägerkorns angereicherten Wirksubstanzen sind im allgemeinen für die zu katalysierende Reaktion verloren. Bei kostbaren Wirksubstanzen, z. B. aus dem Bereich der Edelmetallverbindungen, führt dieser Umstand zu einer finanziellen Belastung der Kontaktkosten pro Einheit hergestelltes Produkt.

Es sind immer wieder Versuche unternommen worden, Katalysatoren herzustellen, bei denen Trägermaterialien mit kleiner BET-Oberfläche die Wirksubstanzen nur in einem schmalen, äußeren Bereich des Trägerkorns enthalten. Von den vielen Versuchen, die unternommen worden sind, sind beispielsweise folgende Maßnahmen zur Herstellung von Trägerkatalysatoren auf Trägern niedriger BET-Oberfläche vorgeschlagen worden:

Imprägnierung von $\alpha$-Al$_2$O$_3$-Trägermaterial mit der wäßrigen Lösung einer Base, partielle Trocknung des so behandelten Trägermaterials auf eine Restfeuchte von 10 bis 90 % der Sättigungsmenge und anschließende Spritzüberziehung mit einer metallsalzhaltigen Lösung (DE-OS 1 944 933);

Basevorbehandlung eines $\gamma$-Al$_2$O$_3$ Trägermaterials zur Ausfällung von Metallverbindungen (z. B. Fe(OH)$_3$) an der Trägeroberfläche, Glühprozeß zur Umwandlung von $\gamma$- in $\alpha$-Al$_2$O$_3$ unter Kornwachstum und Porenvergrößerung, begünstigt durch das ausgefällte Fe(OH)$_3$, und anschließende Imprägnierung mit Edelmetallsalzlösung gemäß DE-OS 2 517 313);

Besprühung von Trägermaterialien mit glatten Oberflächen wie z. B. von Carbonaten, Carbiden etc. mit einer durch Neutralisation einer PdCl$_2$—HCl-Lösung mit NaOH hergestellten kolloidalen PdO·H$_2$O-Lösung, Reifung im Zeitraum von bis zu 4 Tagen und schließlich erneute NaOH-Behandlung (US 3 271 327);

Imprägnierung eines Al$_2$O$_3$-Trägers, der bis zu 0,5 Gew.-% Alkalimetalloxid und bis zu 10 Gew.-% eines Metalloxids als Al-Spinell enthalten kann und durch Glühen von Al$_2$O$_3$ vom Typ Pseudoböhmit hergesellt wird, mit einer Edelmetallsalzlösung, deren Azidität in einem vorgegebenen Verhältnis zum Alkaligehalt des Trägers steht (DE-OS 2 715 094);

Besprühen eines geglühten Al$_2$O$_3$ mit konzentrierter, angesäuerter Pd-Salz-Lösung und anschließen des Glühen (US 2 946 829);

Imprägnierung von OH-gruppenhaltigen Trägern mit einer Lösung von Edelmetall-Amin-Komplexionen, die aus den entsprechenden Edelmetallsalzen durch Zugabe von wäßrigem HN$_4$OH erhalten werden, wobei der Träger gegebenenfalls vorher hydrophobiert wird (DE-OS 2 317 536).

2

Alle diese Verfahren und die dabei erhältlichen Katalysatoren sind jedoch mit erheblichen Nachteilen behaftet. Insbesondere weisen diese Katalysatoren schon auf der Trägeroberfläche eine sehr hohe Edelmetallkonzentration auf. Weiterhin bestehen Unterschiede in der Edelmetallverteilung zwischen den einzelnen Katalysatorteilchen. Ein weiterer Nachteil der vorgeschlagenen Verfahren zur Katalysatorherstellung besteht darin, daß die eingesetzten Trägermaterialien im allgemeinen noch relativ hohe Oberflächen haben, wodurch wiederum Nebenreaktionen mit den Produkten ausgelöst werden können. Weiterhin zeigen diese Katalysatoren in vielen Fällen unzureichende Standzeiten.

Als weiterer Stand der Technik ist die GB-PS 1 283 737 von Interesse. Gemäß dieser wird ein Trägerkatalysator hergestellt, in dem man mindestens eine Metallverbindung auf einem porösen Träger durch »spray coating« aufbringt, indem man den Träger mit der Lösung mindestens einer Metallverbindung in Kontakt bringt, wobei der Träger vorher mit einer alkalischen Lösung imprägniert wurde und zu 25 bis 90% mit Wasser oder Alkohol gesättigt ist. Auf diese Weise werden Katalysatoren erhalten, in denen die Ablagerung der aktiven Komponenten an der Oberfläche des Trägers beginnt und sich ein wesentlicher Teil der aktiven Komponenten auf oder in unmittelbarer Nähe der Oberfläche des Trägers ablagern. Dies geht daraus hervor, daß »spray coating« eine bekannte Methode zur Belegung von Oberflächen ist, daß Flüssigkeitsgehalte des Trägers von 25 bis 90% der Sättigung ein Eindringen der aufgesprühten Metallverbindungen verhindern und, daß der Gehalt der Träger an gelösten Basen beim Aufsprühen mit Metallverbindungen zu einer sofortigen Ausfällung unlöslicher Metallverbindungen führt. Deshalb ist es nicht möglich, nach der in der GB-PS 1 283 737 beschriebenen Arbeitsweise Katalysatoren zu erhalten, welche die Vorteile der erfindungsgemäß hergestellten Trägerkatalysatoren aufweisen.

Die moderne chemische Industrie hat jedoch einen erheblichen Bedarf an immer größeren Mengen Trägerkatalysatoren, die aus Kostengründen möglichst wenig Edelmetall, angereichert in einer engen Ringzone, enthalten und sich durch hohe Selektivitäten und lange Standzeiten auszeichnen sollen.

Es wurde nun ein Verfahren zur Herstellung von Trägerkatalysatoren durch Behandlung von inertem Trägermaterial mit einer Base, anschließendes Trocknen, Aufbringen einer Metallsalzlösung und gegebenenfalls Reduktion zum Metall gefunden, das dadurch gekennzeichnet ist, daß man inertes Trägermaterial mit einer BET-Oberfläche von kleiner als 20 $m^2$/g, mit einer Base entsprechend der Saugfähigkeit des Trägermaterials bis zur Sättigung tränkt, anschließend bis zur Gewichtskonstanz trocknet, in an sich bekannter Weise mit einer Metallsalzlösung ebenfalls bis zur Sättigung tränkt und anschließend gegebenenfalls zum Metall reduziert, wobei man die Base in einer solchen Menge aufbringt, daß 0,01 bis 50 Grammäquivalente Base pro Grammäquivalent Metall von der Tränkung mit der Metallsalzlösung im Träger enthalten sind und den Katalysator mit Wasser mischt, bis keine Ionen der bei der Katalysatorherstellung verwendeten Verbindungen im Waschwasser mehr nachweisbar sind.

Als Trägermaterialien, die mit Basen nach dem erfindungsgemäßen Verfahren vorbehandelt werden, kommen die verschiedensten Systeme, aus denen das inerte Trägermaterial mit einer BET-Oberfläche von kleiner als 20 $m^2$/g, bevorzugt kleiner als 10 $m^2$/g, aufgebaut ist, zur Anwendung. Es handelt sich hierbei im wesentlichen um Metalloxide, Silikate, Spinelle, Carbide, Carbonate usw. und Mischungen derselben. Besonders bevorzugt sind inerte Trägermaterialien wie z. B. Aluminiumoxide, Siliciumdioxide, Siliciumdioxid-Aluminiumoxid-Gemische, amorphe Kieselsäuren, Kieselgure, Barium-, Strontium- oder Calciumcarbonate, Mischungen derselben, gegebenenfalls unter Zusatz von Siliciumdioxiden oder Aluminiumoxiden, Titanoxide, Zirkonoxide, Magnesiumoxide, Magnesiumsilikate, Zirkonsilikate, Magnesium-Aluminium-Spinell, Siliciumcarbide, Wolframcarbide, Mischungen von Siliciumcarbiden mit Siliciumdioxiden oder beliebige Mischungen derselben. Die inerten Träger können in den verschiedensten Formen zur Anwendung kommen, wie z. B. als Kugeln, Granulate, Extrudate, Tabletten, Sattelkörper, Rohrabschnitte, Bruchstücke, Warenkeramik usw.

Die Behandlung des inerten Trägermaterials mit einer Base erfolgt im allgemeinen bei Temperaturen von etwa 5 bis etwa 100°C, bevorzugt bei 10 bis 60°C, besonders bevorzugt bei 20 bis 40°C. Dabei kann die Behandlung bei Normaldruck, bei erhöhten Drücken oder auch bei vermindertem Druck vorgenommen werden. Das Verhältnis von Base zum Metall beträgt 0,01 bis 50, bevorzugt 0,5 bis 20 und besonders bevorzugt 0,5 bis 10 Grammäquivalente Base pro Grammäquivalent Metall. Die Zeit der Einwirkung der Base, die in Form einer wäßrigen oder einer nichtwäßrigen Lösung mit dem Träger zusammengebracht werden kann, ist in weiten Bereichen variierbar und beträgt 5 Minuten bis 72 Stunden.

Die Basen können in den verschiedensten Formen auf die inerten Trägermaterialien zur Einwirkung gebracht werden. Sie können entweder lösungsmittelfrei in flüssiger Form oder als Lösungen im wäßrigen oder nichtwäßrigen Lösungsmittel eingesetzt werden.

Bisweilen kann es zweckmäßig sein, daß das verwendete Lösungsmittel mit der Base eine homogene Phase bildet, was jedoch keineswegs für die Durchführung des erfindungsgemäßen Verfahrens erforderlich ist. Als Lösungsmittel für die Basen kommen beispielsweise in Betracht: Wasser, eine Hydroxylgruppe, Ketogruppe und/oder Carboxylgruppe enthaltende, geradkettige oder verzweigte Kohlenwasserstoffe mit 1 bis 12, bevorzugt mit 1 bis 6 C-Atomen, eine Hydroxylgruppe, Ketogruppe und/oder Carboxygruppe enthaltende cyclische Kohlenwasserstoffe mit 5 bis 7, vorzugsweise 6 Kohlenstoffatomen im Ringsystem und eine Hydroxylgruppe, Ketogruppe und/oder Carboxylgruppe enthaltende heterocyclische Verbindungen mit vorzugsweise 6 Atomen im Ringsystem, die als Heteroatome Sauerstoff und/oder Stickstoff enthalten.

Besonders bevorzugte Lösungsmittel sind Wasser, Alkohole wie Methanol, Äthanol, Propanol, Isopropanol, Butanol, Äthylenglykol, Glyzerin, ferner Ketone wie Aceton und Mischungen derselben, Salzsäure, Schwefelsäure, Salpetersäure, Jodwasserstoffsäure, Bromwasserstoffsäure, Essigsäure und Ameisensäure.

Als Basen, mit denen die inerten Träger nach dem erfindungsgemäßen Verfahren behandelt werden, sind die verschiedensten Verbindungstypen geeignet, die nach den allgemein bekannten Theorien von Brönsted oder von Lewis (s. Lehrbücher der anorganischen Chemie) als solche zu bezeichnen sind. Hiernach sind alle Stoffe als Basen zu bezeichnen, die einerseits $H^+$-Ionen aufnehmen und andererseits über freie Elektronenpaare verfügen. Als Basen kommen beispielsweise in Betracht Oxide, Carbonate, Hydrogencarbonate, Hydrogenphosphate, Hydroxide, Alkoxide, Formiate, Alkalisilikate, Alkalialuminate oder deren Mischungen.

Die in Frage kommenden Alkalisilikate und Alkalialuminate sind beschrieben in Hollemann-Wiberg, »Lehrbuch der anorganischen Chemie«, 71.–80. Auflage, Berlin 1971, Seite 497, 577/578 sowie 583/584.

Für die erfindungsgemäße Trägervorbehandlung mit Basen kommen in Betracht: $Li_2O$, $Na_2O$, $K_2O$, $Rb_2O$, $Cs_2O$, $MgO$, $LiOH$, $NaOH$, $KOH$, $RbOH$, $CsOH$, $Mg(OH)_2$; $Ca(OH)_2$, $Sr(OH)_2$; $Ba(OH)_2$; $Na_2HPO_4$; $Li_2HPO_4$; $K_2HPO_4$; $Na_2CO_3$, $Li_2CO_3$, $K_2CO_3$, $LiOOCCH_3$, $NaOOCCH_3$, $KOOCCH_3$, $NaHCO_3$, $LiHCO_3$, $KHCO_3$; Alkoxide vom Typ MeOR, wobei Me für ein Alkalimetall steht und R einen $C_1 - C_4$-Alkylrest bedeutet, wie z. B. $NaOCH_3$, $NaOC_2H_5$, $NaOC_3H_7$, Alkalisikate vom Typ $ME^I SiO_4$, $Me_6^I Si_2O_7$, $Me_2^I SiO_3$ und $Me_2^I Si_2O_5$ wie z. B. $Na_2SiO_3$, Alkalialuminate wie z. B. $Na[Al(OH)_4]$.

Die Basen können den vorgelegten Trägern zudosiert werden oder umgekehrt. Bevorzugt dosiert man die Lösung der Basen dem vorgelegten Träger zu.

Wesentlicher Bestandteil der Vorbehandlung der inerten Trägermaterialien mit Basen ist die nachfolgende Trocknung in an sich bekannter Art und Weise, beispielsweise diskontinuierlich oder kontinuierlich im Trockenschrank oder Warmluftstrom bei Temperaturen von etwa 50° bis 200°C, vorzugsweise bei 100 bis 150°C. Besonders bevorzugt sind Temperaturen von 105 bis 125°C und Drücke von 1 bar. Das mit der Base beladene Trägermaterial wird so bis zur Gewichtskonstanz getrocknet.

Das auf diese Weise behandelte Trägermaterial wird in einer weiteren Verfahrensstufe entsprechend seiner Saugfähigkeit in an sich bekannter Weise mit einer Metallsalzlösung bis zur Sättigung getränkt. Die Metall- und/oder Edelmetallgehalte der so eingesetzten Lösungen werden derart bemessen, daß entweder durch Einmal- oder durch Mehrfachtränkung die gewünschten Metallkonzentrationen auf den Trägermaterialien eingestellt werden. Sie bestimmen ferner die erforderlichen Mengen an Base, die zur Vorbehandlung der Träger eingesetzt werden. Beispielsweise kommen die Salzlösungen der Metalle der Gruppen IIIa, IVa, Va, VIa, VIIa, VIIIa, Ib, IIb, IIIb, IVb oder Vb des Periodensystems sowie der seltenen Erden als auch der Actiniden zur Anwendung.

Im einzelnen seien beispielsweise folgende Metalle genannt: Sc, Y, Ti, Zr, Hf, V, Nb, Ta, Cr, Mo, W, Mn, Re, Fe, Ru, Os, Co, Rh, Ir, Ni, Pd, Pt, Cu, Ag, Au, Zn, Cd, Hg, Ga, Tl, Ge, Sn, Pb, As, Sb, Bi, Ce, Sm, Th, U. Bevorzugt werden Pd, Ti, Cr, Mn, Fe, Ru, Co, Rh, Ni, Cu, Zn, Ag, Bi, Zr, Ir, Pt, Au, Ce eingesetzt.

Die zur Anwendung kommenden Metalle können in Form ihrer Salze oder Komplexsalze sowohl als Lösung als auch als Suspension eingesetzt werden.

Als Salze und/oder Komplexe dieser Metalle kommen beispielsweise Halogenide, z. B. Fluoride, Chloride, Bromide, Jodide, wie sie in »Halides of the Transition Elements«, Bd. 1 bis 3, von D. Brown, J. H. Canterford und R. Coltan, John Wiley & Sons, Ltd. London 1968, aufgeführt sind, in Betracht, sowie die Verbindungstypen, die in »Structural Inorganic Chemistry« von A. F. Wells, 3. Auflage, Oxford at the Clarendon Press 1967, zusammengestellt sind.

Bevorzugt sind folgende Metall- und Edelmetallsalze zu nennen:

$PbCl_2$, $Pb(NO_3)_2$, $Pb(CH_3COO)_2 \cdot aq$;
$CdCl_2 \cdot H_2O$, $CdJ_2$, $Cd(NO_3)_2 \cdot 4H_2O$, $3CdSO_4 \cdot 8H_2O$, $CeCl_3 \cdot 7H_2O$, $Ce(NO)_3 \cdot 6H_2O$,
$Ce(SO_4)_2 \cdot 4H_2O$, $Cr(NO_3)_3 \cdot 9H_2O$,

$FeCl_2 \cdot 4H_2O$, $FeCl_3 \cdot 6H_2O$, $FeCl_3$, $Fe(NO_3)_3 \cdot 9H_2O$, $FeSO_4 \cdot 7H_2O$, $Fe_2(SO_4)_3 \cdot aq$, $CuBr_2$,
$CuCl_2 \cdot 2H_2O$, $Cu(NO_3)_2 \cdot 3H_2O$, $CuSO_4 \cdot 5H_2O$, $LaCl_3 \cdot 7H_2O$, $La(NO_3)_3 \cdot 6H_2O$,
$MnCl_2 \cdot 4H_2O$, $Mn(NO_3)_2 \cdot 4H_2O$, $MnSO_4 \cdot H_2O$, $NiCl_2 \cdot 6H_2O$, $Ni(NO_3)_2 \cdot 6H_2O$,
$NiSO_4 \cdot 7H_2O$, $ThCl_4$, $Th(NO_3)_4 \cdot 4H_2O$, $TiCl_3$, $TiCl_4$, $TiOSO_4$, $UO_2(NO_3)_2 \cdot 6H_2O$;

$VOSO_4 \cdot 5H_2O$, $VO(C_2O_4) \cdot aq$;
$Bi(NO_3)_3 \cdot 5H_2O$, $Bi(NO_3) \cdot BiO(OH)$, $ZnCl_2$, $Zn(NO_3)_2 \cdot 6H_2O$, $ZnSO_4 \cdot 7H_2O$, $ZrOCl_2 \cdot 8H_2O$,
$H_2[PtCl_6]$, $K_2[PtCl_6]$, $Na_2[PdCl_4]$, $PdCl_2$, $PdBr_2$, $PdJ_2$, $K_2[PdCl_4]$, $Pd(CH_3COO)_2$, $Pd(NO_3)_2$, $PdSO_4$,
$[Pd(NH_3)_2]Cl_2$, $RhCl_3$, $Rh(NO_3)_3$, $H_2[IrCl_6]$, $IrCl_3$, $RuCl_3$, $AgNO_3$, $BiCl_3$, $CrCl_3$, $SmCl_3$, $NbOCl_3$.

Die zum Einsatz kommenden Metallsalzlösungen sind in ihrer chemischen Zusammensetzung von der Art des herzustellenden Katalysators bestimmt. Sie können ein oder mehrere Metalle, gelöst in Form ihrer Salze, enthalten. Physikalische Parameter, wie z. B. Saugfähigkeit des Trägers und Löslichkeit der Metallsalze können zur Erreichung der geforderten Wirkstoffkonzentrationen im fertigen Katalysator Mehrfachtränkungen im Sinne des erfindungsgemäßen Verfahrens erforderlich machen. Beispielsweise wird die Konzentration der Metallsalzlösung so eingestellt, daß der fertige Katalysator 0,5 bis 200 g. bevorzugt 1 bis 100 g einer oder mehrerer katalytisch wirksamer Komponenten pro Liter Träger enthält. Sofern die katalytisch wirksame Komponente ein Edelmetall oder eine Edelmetallverbindung darstellt oder sofern mehrere katalytische Komponenten in dem Trägerkatalysator vorliegen, von denen mindestens eine ein Edelmetall oder eine Edelmetallverbindung darstellt, beträgt der Gehalt dieser Komponenten jeweils 0,5–100 g. bevorzugt 1–50 g. besonders bevorzugt 2–20 g. berechnet als Edelmetall in elementarer Form, pro Liter Träger. Beispielsweise kann der erfindungsgemäße Katalysator 1–20 g. bevorzugt 2–10 g Palladium oder 1–100 g. bevorzugt 2–50 g Silber, oder, im Falle eines Mehrkomponenten-Trägerkatalysators 1–20 g Palladium, 1–50 g Kupfer und 1–50 g Chrom, jeweils berechnet als Metall in elementarer Form, pro Liter Träger enthalten.

Die Möglichkeit einer technischen Durchführung des erfindungsgemäßen Verfahrens sei an Hand der Herstellung eines palladiumhaltigen $\alpha$-Al$_2$O$_3$-Katalysators veranschaulicht.

Das $\alpha$-Al$_2$O$_3$ wird entsprechend seiner Saugfähigkeit mit der Lösung einer Base, z. B. NaOH in H$_2$O, im Verhältnis 0,01 bis 50 Äquivalente Base pro Äquivalent Metall vor der eigentlichen Tränkung mit der Lösung des Metallsalzes vorbehandelt. Danach wird das Trägermaterial bei einer Temperatur von 100 bis 200° C, beispielsweise 110° C, bis zur Gewichtskonstanz betrocknet. Nach an sich bekannten Verfahren wird das so vorbehandelte Trägermaterial entsprechend seiner Saugfähigkeit mit einer Metallsalzlösung, deren Metallgehalt durch die vorher aufgebrachte Basemenge festgelegt ist, beispielsweise Na$_2$[PdCl$_4$], getränkt. Nach einer Reaktionszeit von einigen Stunden bis zu mehreren Tagen, beispielsweise 24 Stunden, werden ein Waschprozeß und eine Trocknung angeschlossen. Falls erforderlich, wird das aufgebrachte Metallsalz nach bekannten Methoden, beispielsweise Behandlung mit N$_2$H$_4$ · H$_2$O-Lösung, zuerst zum Metall reduziert, bevor sich gegebenenfalls ein Waschprozeß und eine Trocknung anschließen.

Der wesentliche Vorteil des erfindungsgemäßen Verfahrens gegenüber den bisher bekannten Verfahren zur Herstellung von Trägerkatalysatoren besteht darin, daß eine ringförmige Anreicherung der Wirksubstanzen im Inneren des inerten Trägermaterials, d. h. direkt unterhalb der Oberfläche, erreicht wird, wobei insbesondere bei den teuren Edelmetallen deutliche Einsparungen erreicht werden. Die so erhaltenen Katalysatoren liefern bei ihrem technischen Einsatz auf Grund der niedrigen Oberfläche des Trägermaterials weniger Nebenprodukte als Trägerkatalysatoren herkömmlicher Art. Weiterhin sind die im Träger angereicherten Wirksubstanzen weitgehend vor Vergiftungen und Verlusten durch Abrieb geschützt. Hierdurch wird eine sehr hohe Lebensdauer der Katalysatoren bei gleichbleibender Aktivität erreicht.

Die erfindungsgemäß hergestellten Trägerkatalysatoren können für die verschiedensten katalytischen Verfahren, wie z. B. Hydrierung, Dehydrierung, Hydrogenolyse, Oxidation, Polymerisation, Isomerisierung oder Cyclisierung verwendet werden. Bei diesen katalytischen Reaktionen können die nach dem erfindungsgemäßen Verfahren hergestellten Trägerkatalysatoren sowohl in der Sumpfphase als auch in der Rieselphase und der Gasphase eingesetzt werden. Bevorzugt sind die Rieselphase und die Gasphase. Dabei kann sowohl unter Normaldruck, Überdruck als auch unter vermindertem Druck gearbeitet werden. Katalytische Hydrierungen sind ein bevorzugtes Anwendungsgebiet für die erfindungsgemäß hergestellten Katalysatoren. Je nach Wirkstoffzusammensetzung eignen sie sich besonders zur Hydrierung aliphatischer Mehrfachbindungen, z. B. für Selektivhydrierungen, zur Kernhydrierung aromatischer Systeme oder zur Hydrierung bestimmter Substituenten, beispielsweise von in aromatischen Systemen enthaltenen Nitro- oder Carbonylgruppen. Bestimmte Wirkstoffzusammensetzungen der nach dem erfindungsgemäßen Verfahren hergestellten Trägerkatalysatoren haben eine bevorzugte Anwendung bei katalytischen Hydrierungen von substituierten Aromaten erlangt, wobei — abhängig von der Kombination der katalytisch wirksamen Substanzen sowie anderer Verfahrensparameter, wie Temperatur oder Druck — entweder das aromatische System und/oder der Substituent hydriert werden können.

So findet beispielsweise die Wirksubstanzkombination Palladium/Vanadium/Blei auf inertem $\lambda$-Al$_2$O$_3$-Träger, der nach dem erfindungsgemäßen Verfahren behandelt wurde, eine bevorzugte Anwendung zur katalytischen Hydrierung von Nitroaromaten, wie z B von Nitrobenzol, Nitrotoluol, Dinitrobenzolen, Dinitrotoluolen, Trinitrotoluolen und Nitrophenolen zu den entsprechenden aromatischen Aminen. Für Mononitroverbindungen wird dabei die Gasphasenreaktion bevorzugt, während für die Dinitro- oder Trinitroverbindungen die Flüssigphase und speziell die Rieselphase bevorzugt wird. Sowohl in der Gasphase als auch in der Rieselphase wird im allgemeinen die zu reduzierende Verbindung über einen fest angeordneten Kontakt geleitet. Es wird vorteilhaft mit einem Wasserstoffüberschuß gearbeitet. Beim Arbeiten in der Rieselphase wird die zu reduzierende Nitroverbindung gewöhnlich mit der bei der Reduktion entstehenden Aminoverbindung oder einem anderen Verdünnungsmittel so weit verdünnt, daß eine Durchführung der Reduktion ungefährlich ist. Die bevorzugte Reaktionstemperatur in der Rieselphase liegt im Bereich bei 50 bis 150° C und der bevorzugte Druckbereich zwischen 1 und 100 bar.

Bei der Hydrierung in der Gasphase wird bevorzugt im Temperaturbereich von 150 bis 350° C und bei 1 bis 10 bar gearbeitet.

Ein nach dem erfindungsgemäßen Verfahren durch Abscheidung von Palladium auf inertem $\alpha$-Al₂O₃ hergestellter Katalysator findet wiederum eine besondere Anwendung bei der Hydrierung von Phenol oder von m/p-Kresol zu Cyclohexanon bzw. m/p-Methylcyclohexanon. Hierbei wird vorteilhaft in der Gasphase hydriert. Der bevorzugte Temperaturbereich liegt bei 100 bis 200° C. Es wird üblicherweise drucklos oder unter geringfügigem Überdruck von 0,1 bis 1 bar gearbeitet.

Die mit erfindungsgemäß hergestellten Trägerkatalysatoren durchgeführten katalytischen Reaktionen zeichnen sich durch hohe Selektivität und geringe Nebenproduktbildung aus. Die gleichbleibende Katalysatoraktivität bewirkt lange Laufzeiten.

Beispiele

A) Beschreibung der eingesetzten Trägermaterialien

Tabelle 1

| Träger Nr. | Träger Zusammensetzung | Geometrische Form | BET m²/g | Schütt- gew. g/l | H₂O-Auf- nahme ml/100 g |
|---|---|---|---|---|---|
| 1 | Al₂O₃ < 5% SiO₂ | Extrudat 3 mm ⌀ | 3,1 | 1161 | 28,0 |
| 2 | Al₂O₃ < 5% SiO₂ | Extrudat 5 mm ⌀ | 6,0 | 1075 | 26,4 |
| 3 | Al₂O₃ < 5% SiO₂ | Extrudat 2 mm ⌀ | 9,0 | 962 | 32,0 |
| 4 | Al₂O₃ | Kugeln 3—6 mm | 9,8 | 812 | 45,1 |
| 5 | Al₂O₃ | Tabletten 5 × 5 mm | 8,5 | 562 | 85,0 |
| 6 | Al₂O₃ ~ 5% Li₂O | Kugeln 4—5,5 mm | 20,0 | 770 | 44,0 |
| 7 | Al-Silikat ~ 88% SiO₂ ~ 12% Al₂O₃ | Granulat 3,5—4,5 mm | 1,8 | 760 | 32,3 |
| 8 | Al-Silikat ~ 85% Al₂O₃ ~ 15% SiO₂ | Granulat 3—5 mm ⌀ | 4,5 | 1190 | 22,4 |
| 9 | Al-Silikat ~ 85% Al₂O₃ > 10% SiO₂ | Kugeln 4—5 mm ⌀ | 0,1 | 932 | 12,6 |
| 10 | Mg-Al-Silikat ~ 53% Al₂O₃ ~ 32% SiO₂ ~ 15% MgO | Kugeln 6 mm ⌀ | 2,1 | 1060 | 21,0 |
| 11 | Al-Silikat ~ 88% SiO₂ ~ 12% Al₂O₃ | Kugeln 8 mm ⌀ | 1,0 | 1000 | 28,6 |
| 12 | Zr-Mg-Silikat ~ 88% SiO₂ ~ 6% ZrO₂ ~ 3% MgO Rest Al₂O₃ | Kugeln 8 mm ⌀ | 5,6 | 1050 | 12,4 |
| 13 | SiO₂ < 5% Al₂O₃ | Kugeln 5 mm ⌀ | 10,0 | 834 | 27,0 |
| 14 | SiO₂ | Tabletten 3 × 3 mm | 6,5 | 1132 | 11,0 |
| 15 | Al-Silikat ~ 85% SiO₂ ~ 15% Al₂O₃ | Tabletten 4 × 4 mm | 3,9 | 1198 | 9,0 |

6

0 012 153

B) Herstellung der Katalysatoren

Beispiel 1

a) Allgemeine Beschreibung der Katalysatorherstellung

1000 ml eines Trägers aus Tabelle 1 werden bei Raumtemperatur mit einer die erforderliche Menge Base enthaltenden Lösung in einer rotierenden Trommel bis zur Sättigung imprägniert.

Das Volumen der Imprägnierlösung wird über die Saugfähigkeit und das Schüttgewicht des Trägers berechnet.

Die dementsprechend eingesetzte Imprägnierlösung wird von dem jeweiligen Träger innerhalb weniger Minuten völlig aufgesaugt. Der so imprägnierte Träger wird in einer rotierenden Trommel oder in einem anderen geeigneten Gefäß in einem warmen, gegebenenfalls inerten Gasstrom bis zu 200° C bis zur Gewichtskonstanz getrocknet.

Der Restfeuchtegehalt des getrockneten, baseimprägnierten Trägers muß nach Abkühlen auf Raumtemperatur in einem Bereich von weniger als 10% der Sättigungsmenge des Trägers liegen. Der so vorbehandelte, trockene Träger wird erneut entsprechend seiner Saugfähigkeit mit einer, die erforderliche Menge Wirkstoff enthaltenden Lösung, wie oben beschrieben, imprägniert. Nach dieser Imprägnierung wird der feuchte Träger in ein geeignetes, verschließbares Gefäß gefüllt und entsprechend einer Reifezeit bis zu mehreren Tagen darin belassen. Der so behandelte Träger wird anschließend mit Wasser gewaschen und/oder getrocknet.

Falls erforderlich, werden die auf dem Träger abgeschiedenen Wirkstoffe nach allgemein bekannten Methoden reduziert. Der Katalysator wird anschließend mit Wasser gewaschen und den Katalysator mit Wasser mischt, bis keine Ionen der bei der Katalysatorherstellung verwendeten Verbindungen im Waschwasser mehr nachweisbar sind und gegebenenfalls getrocknet.

b) Einzelbeispiel

Es werden z. B. 1000 ml des Trägers Nr. 1 aus Tabelle 1 entsprechend 1161 g bei einer Saugfähigkeit von 28 ml Wasser pro 100 g-Träger mit 1,2 g = 0,03 g-Äquivalent Natronlauge, aufgelöst in 325 ml Wasser, imprägniert. Die Imprägnierlösung wird innerhalb von 2 Minuten völlig von dem Träger aufgesaugt. Der feuchte Träger wird in ein entsprechend dimensioniertes, zylindrisches Gefäß umgefüllt, im Warmluftstrom von 110°C bis zur Gewichtskonstanz und auf einen Restfeuchtegehalt von 0,11 Gew.-% entsprechend ca. 0,39% der Sättigungsmenge getrocknet.

Der so vorbehandelte Träger wird anschließend erneut mit dem seiner Saugfähigkeit bis zur Sättigung entsprechenden Volumen einer wäßrigen Lösung, enthaltend 2,0 g = 0,037 g-Äquivalent Palladium in Form von Natriumtetrachloropalladat-(II), imprägniert und feucht in ein entsprechend dimensioniertes, verschließbares Gefäß umgefüllt.

Die im Träger enthaltene Menge NaOH entspricht einem Äquivalenzverhältnis [g-Äquivalent NaOH : g-Äquivalent Pd] von 0,81. Nach einer Reaktionszeit von 15 Minuten wird der mit Natriumtetrachloropalladat-(II) imprägnierte, mit Natronlauge vorbehandelte Träger Nr. 1 in dem Aufbewahrungsgefäß mit 400 ml einer wäßrigen, 10% Hydrazinhydrat enthaltenden Lösung überschichtet, wodurch die auf dem Träger abgeschiedene Palladiumverbindung zum metallischen Palladium reduziert wird. Nach einer Reaktionszeit von 2 Stunden wird die Reduktionslösung abdekantiert. Der Katalysator wird mit Wasser gewaschen bis kein Reduktionsmittel und keine Ionen der bei der Katalysatorherstellung verwendeten Verbindungen im Waschwasser mehr nachweisbar sind. Die Trocknung des Katalysators erfolgt wie oben beschrieben im Warmluftstrom von 110°C. Der so hergestellte Katalysator enthält 2 g Palladium pro Liter Träger (ca. 0,172 Gew.-%) in einer im Inneren und dicht unter der Oberfläche des Trägers liegenden Ringzone.

Die nach dem erfindungsgemäßen Verfahren hergestellten Katalysatoren wurden zur Ausprüfung ihrer katalytischen Eigenschaften wie folgt getestet:

c) Austestung in der Flüssigphase in einer Laborhydrierapparatur

Die Ausprüfung der katalytischen Aktivität von Katalysatoren wird nach einer standardisierten Methode der Hydrierung von o-Nitrotoluol in methanolischer Lösung in einer Laborhydrierapparatur durchgeführt. Die hierzu verwendete Apparatur entspricht in ihrem wesentlichen Aufbau den in der Literatur bekannten Ausführungen von Schüttel- oder Rührapparaturen, wie sie z. B. in »Methoden der organischen Chemie« (Houben-Weyl) Band IV/2, Georg Thieme Verlag, Stuttgart, 1955, und in der Fachzeitschrift für das Laboratorium, G-I-T Verlag, Darmstadt, in den Ausgaben September 1965 und Oktober 1974 beschrieben sind.

Zur Austestung werden 10 ml Katalysator zur Hydrierung von 5 g o-Nitrotoluol (Merck, 98%ig) in 50 ml Methanol (Merck, 99%ig) bei einer konstanten Hydriertemperatur von 50°C und einem konstanten Druck von 1000 mm Wassersäule eingesetzt. Die Hydrierung erfolgt in einem Glasgefäß

7

mit einem Nutzinhalt von 100 ml durch vertikale Schüttelbewegung von konstantem Hub (205 Hübe/Minute, Hubhöhe 80 mm). Als Maß für die Hydrieraktivität des jeweils eingesetzten Katalysators gilt die Geschwindigkeit der Wasserstoffaufnahme, die durch automatische, graphische Darstellung den zeitlichen Verlauf der Hydrierung wiedergibt. Die unter gleichen Bedingungen und in der gleichen Apparatur erhältlichen Hydrierergebnisse sind sehr gut reproduzierbar und können zur Beurteilung der katalytischen Aktivität der eingesetzten Katalysatoren untereinander verwendet werden.

Sie stellen allerdings keine absoluten Werte dar, da bei einer Katalysatorausprüfung in einer Laborhydrierapparatur der apparatespezifische Faktor der verwendeten Hydrierapparatur berücksichtigt werden muß. Daher können bei einer Katalysatortestung in einer anderen Hydrierapparatur, wie z. B. in dem in der G-I-T Fachzeitschrift für das Laboratorium, Heft 10 Oktober 1974, Seite 974 bis 976, G-I-T Verlag Ernst Giebeler, Darmstadt, beschriebenen Niederdruck-Hydrierapparat, System Roche-Kühner Typ NDH, durchaus andere Hydrierergebnisse erhalten werden, wobei aber die zu erwartenden Aktivitätsunterschiede in den gleichen Abstufungen liegen.

Das Ergebnis der Aktivitätsprüfung des gemäß Beispiel 1 hergestellten Katalysators ist in Tabelle 7 aufgeführt. Es ist ersichtlich, daß die Hydrierung nach einer Hydrierdauer von 54 Minuten beendet war und daß nach dieser Zeit 100% des eingesetzten o-Nitrotoluols umgesetzt wurden.


Beispiel 2 bis 21

Einsatz inerter Al₂O₃-Träger Nr. 1 bis 5 aus Tabelle 1

Entscheidend Beispiel 1 a) wurden Al₂O₃-Träger einer NaOH-Vorbehandlung mit anschließender Palladium-Beladung gemäß Tabelle 2 unterworfen:

Tabelle 2

| Beispiel Nr | Vorbehandlung von je 1000 ml Träger mit wäßriger NaOH-Lösung | | | | | Restfeuchte nach Trockng. | Beladung des behandelten Trägers mit Palladium in Form von wäßriger Na$_2$(PdCl$_4$)-Lösung Palladium-Einsatz | | g-Äquivalent NaOH pro g-Äquivalent Palladium |
|---|---|---|---|---|---|---|---|---|---|
| | Träger-Einsatz | | NaOH-Einsatz | | | | | | |
| | Nr | g | g | g-Äquivalent | ml Lösg | % der Sättigung | g | g-Äquivalent | |
| 2 | 1 | 1161 | 1,8 | 0,045 | 325 | 0,4 | 3 | 0,056 | 0,80 |
| 3 | 1 | 1161 | 6,0 | 0,150 | 325 | 0,9 | 3 | 0,056 | 2,68 |
| 4 | 2 | 1075 | 0,2 | 0,005 | 284 | 0,3 | 2 | 0,037 | 0,14 |
| 5 | 2 | 1075 | 0,4 | 0,010 | 284 | 0,6 | 2 | 0,037 | 0,27 |
| 6 | 2 | 1075 | 0,8 | 0,020 | 284 | 0,4 | 2 | 0,037 | 0,54 |
| 7 | 2 | 1075 | 1,2 | 0,030 | 284 | 0,3 | 2 | 0,037 | 0,81 |
| 8 | 2 | 1075 | 1,6 | 0,040 | 284 | 0,5 | 2 | 0,037 | 1,08 |
| 9 | 2 | 1075 | 2,0 | 0,050 | 284 | 0,7 | 2 | 0,037 | 1,35 |
| 10 | 2 | 1075 | 2,4 | 0,060 | 284 | 0,6 | 2 | 0,037 | 1,62 |
| 11 | 2 | 1075 | 4,0 | 0,100 | 284 | 0,7 | 2 | 0,037 | 2,70 |
| 12 | 3 | 962 | 1,2 | 0,030 | 308 | 0,3 | 2 | 0,037 | 0,81 |
| 13 | 4 | 812 | 1,2 | 0,030 | 366 | 0,2 | 2 | 0,037 | 0,81 |
| 14 | 4 | 812 | 3,0 | 0,075 | 366 | 0,3 | 2 | 0,037 | 2,03 |
| 15 | 4 | 812 | 7,5 | 0,188 | 366 | 0,7 | 2 | 0,037 | 5,08 |
| 16 | 4 | 812 | 3,0 | 0,075 | 366 | 0,3 | 5 | 0,094 | 0,80 |
| 17 | 4 | 812 | 7,5 | 0,188 | 366 | 0,7 | 5 | 0,094 | 2,00 |
| 18 | 4 | 812 | 10,8 | 0,270 | 366 | 0,9 | 9 | 0,169 | 1,60 |
| 19 | 4 | 812 | 43,2 | 1,080 | 366 | 1,3 | 9 | 0,169 | 6,39 |
| 20 | 4 | 812 | 86,4 | 2,160 | 366 | 2,1 | 9 | 0,169 | 12,78 |
| 21 | 5 | 562 | 6,0 | 0,150 | 478 | 0,2 | 5 | 0,094 | 1,60 |

Die so mit Palladium beladenen Träger enthalten jeweils das Palladium in ein und derselben Verteilung innerhalb einer engbegrenzten, im Inneren und dicht unter der Oberfläche des Trägers liegenden Zone.

Beispiele 22 bis 29

Vergleichsbeispiele ohne NaOH-Vorbehandlung der Träger Nr. 1 bis 4 aus Tabelle 1

In Anlehnung an Beispiel 1 a), jedoch ohne NaOH-Vorbehandlung des Trägers, wurden gemäß Tabelle 3 jeweils 1000 ml Träger mit dem seiner Saugfähigkeit bis zur Sättigung entsprechenden Volumen einer wäßrigen, Natriumtetrachloropalladat (II) enthaltenden Lösung imprägniert und anschließend nach einer Reaktionszeit von 15 Minuten mit Hydrazinhydrat reduziert, mit Wasser gewaschen und getrocknet

Tabelle 3

| Beispiel Nr. | Träger-Einsatz | | Beladung des Trägers mit Palladium in Form von wäßriger Na₂[PdCl₄]-Lösung | | |
|---|---|---|---|---|---|
| | Nr. | g | g Pd | g-Äquivalent Pd | ml Losung |
| 22 | 1 | 1161 | 2 | 0,037 | 325 |
| 23 | 1 | 1161 | 3 | 0,056 | 325 |
| 24 | 2 | 1075 | 2 | 0,037 | 284 |
| 25 | 2 | 1075 | 5 | 0,094 | 284 |
| 26 | 3 | 962 | 2 | 0,037 | 308 |
| 27 | 4 | 812 | 2 | 0,037 | 366 |
| 28 | 4 | 812 | 5 | 0,094 | 366 |
| 29 | 4 | 812 | 9 | 0,169 | 366 |

Die so mit Palladium ohne NaOH-Vorbehandlung beladenen Träger enthalten jeweils das Palladium in einer inhomogenen und teilweise über das ganze Trägerkorn verteilten Form.

## Beispiel 30

### Vergleichsbeispiel zum Stand der Technik am Träger Nr. 6 aus Tabelle 1

Geht man von einem in der DE-OS 2 135 155 beschriebenen Spinell-Träger, der dem Träger Nr. 6 aus Tabelle 1 entspricht, aus, um diesen mit Palladium zu belegen, so wird auf Grund der Alkalität des Trägers keine NaOH-Vorbehandlung durchgeführt. Der Spinell-Träger enthält nämlich wasserlösliches $Li_2O$, daß verfahrensbedingt in unterschiedlichen Mengen in den einzelnen Trägerkörnern verteilt ist.

Bei der Imprägnierung von 1000 ml Träger Nr. 6 mit 9 g Pd in Form von $Na_2[PdCl_4]$, gelöst in 339 ml Wasser, und anschließender Reduktion mit wäßriger Hydrazinhydrat-Lösung, Waschung und Trocknung wird ein Katalysator erhalten, dessen Palladiumverteilung in den einzelnen Katalysatorkugeln alle Übergänge zwischen ringförmiger Palladiumabscheidung an der Katalysatoroberfläche bis zur völligen Durchtränkung bis ins Kugelinnere aufweist.

## Beispiele 31 bis 39

### Einsatz anderer inerter Träger 7 bis 15 aus Tabelle 1

Entsprechend Beispiel 1 a) wurden die Träger Nr. 7 bis 15 gemäß Tabelle 4 einer NaOH-Vorbehandlung mit anschließender Palladium-Beladung unterworfen:

Tabelle 4

| Bei- spiel Nr. | Vorbehandlung von je 1000 ml Träger mit wäßriger NaOH Lösung | | | | | Rest feuchte nach Trockng | Beladung des behan- delten Trägers mit Palladium in Form von wäßriger Na₂(PdCl₄)-Lösung | | g-Àqui- valent NaOH pro g-Äqui- valent Palladium |
|---|---|---|---|---|---|---|---|---|---|
| | Träger- Einsatz | | NaOH-Einsatz | | | | | Palladium-Einsatz | |
| | Nr. | g | g | g-Äqui- valent | ml Lösg | % der Sättigung | g | g-Àqui- valent | |
| 31 | 7 | 760 | 3,0 | 0,075 | 245 | 0,4 | 5 | 0,094 | 0,80 |
| 32 | 8 | 1190 | 3,0 | 0,075 | 267 | 0,6 | 5 | 0,094 | 0,80 |
| 33 | 9 | 932 | 6,0 | 0,150 | 117 | 0,8 | 5 | 0,094 | 1,60 |
| 34 | 10 | 1060 | 12,0 | 0,300 | 223 | 0,3 | 10 | 0,187 | 1,60 |
| 35 | 11 | 1000 | 12,0 | 0,300 | 286 | 0,4 | 10 | 0,187 | 1,60 |
| 36 | 12 | 1050 | 6,0 | 0,150 | 130 | 0,7 | 5 | 0,094 | 1,60 |
| 37 | 13 | 834 | 7,5 | 0,188 | 225 | 0,5 | 10 | 0,187 | 1,01 |
| 38 | 14 | 1132 | 12,0 | 0,300 | 125 | 0,8 | 10 | 0,187 | 1,60 |
| 39 | 15 | 1198 | 7,5 | 0,188 | 108 | 0,8 | 5 | C,094 | 2,00 |

### Beispiele 40 bis 50

Einsatz anderer Basen als NaOH zur Trägervorbehandlung von Trägern aus Tabelle 1

Entsprechend Beispiel 1 a) wurden die Träger gemäß Tabelle 5 einer Basevorbehandlung mit anschließender Palladium-Beladung unterworfen, wobei in jedem Fall der Träger vor der Palladium-Beladung mit einer anderen Base als NaOH behandelt wurde.

Tabelle 5

| Bei-spiel Nr. | Vorbehandlung von je 1000 ml Träger mit einer wäßrigen Lösung einer Base | | | | | | Rest-feuchte nach Trockng. | Beladung des behan-delten Trägers mit Palladium in Form von wäßriger $Na_2(PdCl_4)$-Lösung Palladium-Einsatz | | g-Äquivalent Base pro g Aquivalent Palladium |
|---|---|---|---|---|---|---|---|---|---|---|
| | Träger-Einsatz | | Base-Einsatz | | | | | | | |
| | Nr. | g | Base | g | g-Äqui-valent | ml Lösg. | % der Sättigung | g | g-Äqui-valent | |
| 40 | 1 | 1161 | $Na_2CO_3$ | 3,0 | 0,057 | 325 | 0,2 | 2 | 0,037 | 1,54 |
| 41 | 2 | 1075 | $Na_2H\,PO_4 \cdot 12\,H_2O$ | 13,5 | 0,075 | 284 | 0,4 | 2 | 0,037 | 2,03 |
| 42 | 2 | 1075 | $KHCO_3$ | 3,8 | 0,038 | 284 | 0,2 | 2 | 0,037 | 1,03 |
| 43 | 2 | 1075 | $NaC_2H_3O_2 \cdot 3\,H_2O$ | 5,0 | 0,037 | 284 | 0,3 | 2 | 0,037 | 1,00 |
| 44 | 2 | 1075 | $Ba(C_2H_3O_2)_2H_2O$ | 10,0 | 0,073 | 284 | 0,4 | 2 | 0,037 | 1,97 |
| 45 | 2 | 1075 | $Mg(C_2H_3O_2)_2 \cdot 4\,H_2O$ | 8,0 | 0,075 | 284 | 0,4 | 2 | 0,037 | 2,03 |
| 46 | 4 | 812 | KOH | 10,5 | 0,187 | 366 | 0,3 | 5 | 0,094 | 1,99 |
| 47 | 4 | 812 | LiOH (98%) | 4,5 | 0,184 | 366 | 0,3 | 5 | 0,094 | 1,96 |
| 48 | 4 | 812 | $NaHCO_3$ | 8,0 | 0,095 | 366 | 0,4 | 5 | 0,094 | 1,01 |
| 49 | 4 | 812 | $K_2CO_3$ | 13,0 | 0,188 | 366 | 0,2 | 5 | 0,094 | 2,00 |
| 50 | 4 | 812 | $HCO_2Na$ | 23,0 | 0,339 | 366 | 0,3 | 9 | 0,169 | 2,00 |

### Beispiele 51 bis 67

### NaOH-Vorbehandlung von Träger Nr. 4 aus Tabelle 1 mit anschließender Beladung mit anderen Wirkstoffen als Palladium und Kombinationen derselben einschließlich Palladium

Entsprechend Beispiel 1 a) wurden für jeden Ansatz 1000 ml = 812 g Träger Nr. 4 eingesetzt und gemäß Tabelle 6 mit dem seiner Saugfähigkeit bis zur Sättigung entsprechenden Volumen von 366 ml einer wäßrigen, NaOH enthaltenden Lösung imprägniert, getrocknet und anschließend einer Beladung mit Wirkstoffen unterworfen.

Tabelle 6

| Bei-spiel Nr. | Vorbehandlung von 1000 ml = 812 g Träger Nr. 4 mit 365 ml wäßriger NaOH-Lsg. NaOH-Einsatz | | Rest-feuchte nach Trock-nung | Beladung des behandelten Trägers mit Wirkstoffen in Form ihrer Salze in wäßriger Lösung Wirkstoff-Einsatz Salz | | Metall | | Summe g-Äqui-valent | g-Äquivalent NaOH pro g-Äquivalent Gesamt-metalle |
|---|---|---|---|---|---|---|---|---|---|
| | g | g-Äqui-valent | % der Sättig. | g | Salz | g | g-Äqui-valent | | |
| 51 | 30 | 0,750 | 0,7 | 71,2 | $FeCl_2 \cdot 4\,H_2O$ | 20 | 0,716 | 0,716 | 1,05 |
| 52 | 30 | 0,750 | 0,7 | 81,0 | $NiCl_2 \cdot 6\,H_2O$ | 20 | 0,681 | 0,681 | 1,10 |
| 53 | 30 | 0,750 | 0,7 | 80,8 | $CoCl_2 \cdot 6\,H_2O$ | 20 | 0,679 | 0,679 | 1,11 |
| 54 | 30 | 0,750 | 0,7 | 45,8 | $MnCl_2$ | 20 | 0,728 | 0,728 | 1,03 |
| 55 | 26 | 0,650 | 0,6 | 24,9 | $Na[PdCl_4]$ | 9 | 0,169 | 0,527 | 1,23 |
| | | | | 35,6 | $FeCl_2 \cdot 4\,H_2O$ | 10 | 0,358 | | |
| 56 | 26 | 0,650 | 0,6 | 24,9 | $Na_2[PdCl_4]$ | 9 | 0,169 | 0,510 | 1,27 |
| | | | | 40,5 | $NiCl_2 \cdot 6\,H_2O$ | 10 | 0,341 | | |
| 57 | 26 | 0,650 | 0,6 | 24,9 | $Na_2[PdCl_4]$ | 9 | 0,169 | 0,509 | 1,28 |
| | | | | 40,4 | $CoCl_2 \cdot 6\,H_2O$ | 10 | 0,340 | | |
| 58 | 26 | 0,650 | 0,6 | 24,9 | $Na_2[PdCl_4]$ | 9 | 0,169 | 0,533 | 1,22 |
| | | | | 22,9 | $MnCl_2$ | 10 | 0,364 | | |
| 59 | 45 | 1,125 | 0,9 | 24,9 | $Na_2[PdCl_4]$ | 9 | 0,169 | 0,867 | 1,30 |
| | | | | 24,2 | $CuCl_2 \cdot 2\,H_2O$ | 9 | 0,283 | | |
| | | | | 36,9 | $CrCl_3 \cdot 6\,H_2O$ | 7,2 | 0,415 | | |
| 60 | 80 | 2,00 | 1,9 | 24,9 | $Na_2[PdCl_4]$ | 9 | 0,169 | 1,567 | 1,28 |
| | | | | 48,3 | $CuCl_2 \cdot 2\,H_2O$ | 18 | 0,567 | | |
| | | | | 73,8 | $CrCl_3 \cdot 6\,H_2O$ | 14,4 | 0,831 | | |
| 61 | 11 (10)*) | 0,275 | 0,4 | 24,9 | $Na_2[PdCl_4]$ | 9 | 0,169 | 0,212 | 1,30 |
| | | | | 4,5 | $BiCl_3$ (gelöst in HCl) | 3 | 0,043 | | |
| 62 | 11 (20)*) | 0,275 | 0,4 | 24,9 | $Na_2[PdCl_4]$ | 9 | 0,169 | 0,255 | 1,08 |
| | | | | 9,0 | $BiCl_3$ (gelöst in HCl) | 6 | 0,086 | | |

Fortsetzung

| Bei-spiel Nr. | Vorbehandlung von 1000 ml = 812 g Träger Nr. 4 mit 365 ml wäßriger NaOH-Lsg. NaOH-Einsatz | | Rest-feuchte nach Trock-nung | Beladung des behandelten Trägers mit Wirkstoffen in Form ihrer Salze in wäßriger Lösung Wirkstoff-Einsatz Salz | | Metall | | | g-Äquivalent NaOH pro g-Aquivalent Gesamt-metalle |
| | g | g-Äqui-valent | % der Sättig. | g | Salz | g | g-Äqui-valent | Summe g-Äqui-valent | |
|---|---|---|---|---|---|---|---|---|---|
| 63 | 10 | 0,250 | 0,4 | 16,6 6,1 | $Na_2[PdCl_4]$ $Na[AuCl_4] \cdot 2 H_2O$ | 6 3 | 0,112 0,046 | 0,158 | 1,58 |
| 64 | 8 | 0,200 | 0,3 | 25,3 | $Th(NO_3)_4 \cdot 6 H_2O$ | 10 | 0,172 | 0,172 | 1,16 |
| 65 | 10 | 0,250 | 0,4 | 24,3 | $SmCl_3 \cdot 6 H_2O$ | 10 | 0,199 | 0,199 | 1,26 |
| 66 | 50 | 1,250 | 1,3 | 32,3 | $TiCl_3 \cdot$ (15%ige Lösung) | 10 | 0,626 | 0,626 | 2,00 |
| 67 | 20 | 0,500 | 0,5 | 78,7 | $AgNO_3$ | 50 | 0,463 | 0,463 | 1,08 |

*) Werte in Klammern entsprechen der NaOH-Menge, die zusätzlich zur Neutralisation der in der $BiCl_3$-Lösung enthaltenen HCl eingesetzt wurde.

### Beispiel 68

Einsatz nichtwäßriger Lösungen zur Basevorbehandlung des Trägers und
zur Belegung des vorbehandelten Trägers mit Wirksubstanzen

Entsprechend der Arbeitsweise von Beispiel 1 a) wurde ein weiterer Katalysator mit der Abänderung hergestellt, daß 1000 ml Träger Nr. 4, Tabelle 1, mit 28 g = 0,519 g-Äquivalent Natriummethylat, gelöst in 366 ml Methanol, imprägniert, getrocknet und anschließend mit 40,5 g = 0,341 g-Äquivalent Nickel(II)-Chlorid − 6 Hydrat, gelöst in 366 ml Methanol, erneut imprägniert und getrocknet wurden.

Die im Träger enthaltene Menge Natriummethylat entsprach einem Äquivalenzverhältnis (g-Äquivalent $CH_3ONa$ zu g-Äquivalent Ni) von 1,52.

Der so hergestellte Katalysator enthält die Wirksubstanz in einer im Inneren und dicht unter der Oberfläche des Trägers liegenden Ringzone.

### Beispiel 69

Beladung eines Pd-haltigen Katalysators mit weiteren Wirksubstanzen

1000 ml des gemäß Beispiel 18, Tabelle 2, hergestellten und 9 g Palladium pro Liter Träger enthaltenden Katalysators wurden mit einer wäßrigen, 9 g Vanadium in Form von Vanadyloxalat enthaltenden Lösung imprägniert und getrocknet. Die bisher auf dem Träger aufgebrachten Wirkstoffe wurden anschließend einer thermischen Behandlung mit Luft unterworfen. Nach dieser Behandlung wurde der so erhaltene Katalysator erneut mit einer wäßrigen, 3 g Blei in Form von Bleiacetat enthaltenden Lösung imprägniert und getrocknet.

Beispiel 70

Ausprüfung der erfindungsgemäß hergestellten Katalysatoren
in der Laborhydrierapparatur gemäß Beispiel 1c)

Tabelle 7

| Beispiel Nr. | aus Tabelle Nr. | Träger Nr. | Pd-Gehalt g/l | g-Äquivalent NaOH pro g-Äquivalent Pd | Hydrier- dauer Minuten | Umsatz o-Nitrotoluol % |
|---|---|---|---|---|---|---|
| 1 | | 1 | 2 | 0,81 | 54 | 100 |
| 22 Vergleich | 3 | 1 | 2 | 0,00 | 60 | 55 |
| 2 | 2 | 1 | 3 | 0,80 | 41 | 100 |
| 3 | 2 | 1 | 3 | 2,68 | 33 | 100 |
| 23 Vergleich | 3 | 1 | 3 | 0,00 | 60 | 92 |
| 5 | 2 | 2 | 2 | 0,27 | 50 | 100 |
| 6 | 2 | 2 | 2 | 0,54 | 43 | 100 |
| 10 | 2 | 2 | 2 | 1,62 | 36 | 100 |
| 24 Vergleich | 3 | 2 | 2 | 0,00 | 56 | 100 |
| 25 Vergleich | 3 | 2 | 5 | 0,00 | 41 | 100 |
| 12 | 2 | 3 | 2 | 0,81 | 46 | 100 |
| 26 Vergleich | 3 | 3 | 2 | 0,00 | 60 | 77 |
| 13 | 2 | 4 | 2 | 0,81 | 60 | 35 |
| 14 | 2 | 4 | 2 | 2,03 | 55 | 100 |
| 15 | 2 | 4 | 2 | 5,08 | 41 | 100 |
| 27 Vergleich | 3 | 4 | 2 | 0,00 | 60 | 35 |
| 16 | 2 | 4 | 5 | 0,80 | 46 | 100 |
| 17 | 2 | 4 | 5 | 2,00 | 31 | 100 |
| 28 Vergleich | 3 | 4 | 5 | 0,00 | 52 | 100 |
| 18 | 2 | 4 | 9 | 1,60 | 32 | 100 |
| 19 | 2 | 4 | 9 | 6,39 | 27 | 100 |
| 20 | 2 | 4 | 9 | 12,78 | 20 | 100 |
| 29 Vergleich | 3 | 4 | 9 | 0,00 | 39 | 100 |

Die in Tabelle 7 aufgeführten Hydrierergebnisse zeigen deutlich, daß die nach dem erfindungsgemäßen Verfahren durch Base-Vorbehandlung der Träger hergestellten Katalysatoren eine wesentlich höhere Hydrieraktivität besitzen als die nach herkömmlicher Methode ohne

17

Base-Vorbehandlung der Träger hergestellten Vergleichskatalysatoren. Es ist klar ersichtlich, daß z. B. der nach dem erfindungsgemäßen Verfahren gemäß Beispiel 15 hergestellte Katalysator mit 2 g Pd pro Liter Träger eine mehr als 4fach höhere Aktivität besitzt als der mit gleichem Pd-Gehalt gemäß Beispiel 27 hergestellte Vergleichskatalysator.

Es ist weiterhin klar ersichtlich, daß der erfindungsgemäß nach Beispiel 15 hergestellte Katalysator mit nur 2 g Pd pro Liter Träger noch eine bessere Aktivität besitzt als der gemäß Beispiel 28 hergestellte Vergleichskatalysator mit einem Pd-Gehalt von 5 g Pd pro Liter Träger. Außerdem ist zu erkennen, daß dieser nach dem erfindungsgemäßen Verfahren hergestellte, 2 g Pd pro Liter enthaltende Katalysator fast die Hydrieraktivität des gemäß Beispiel 29 hergestellten, 9 g Pd pro Liter enthaltenden Vergleichskatalysator erreicht.

### Beispiel 71

### Technische Hydrierung von o-Nitrotoluol

Ein Rohrreaktor (Rohrlänge 6 m; Rohrdurchmesser 24 mm) wurde mit 2,4 Liter des nach Beispiel 69 hergestellten Katalysators, der, als Metall berechnet, 9 g Pd, 9 g V und 3 g Pb pro Liter $\alpha$-Al$_2$O$_3$-Träger enthielt, gefüllt. Bei Raumtemperatur und ca. 50 bar Wasserstoffdruck wurde das zu hydrierende o-Nitrotoluol (4 Teile) im Gemisch mit o-Toluidin (6 Teile) mit 1 kg/h · Liter Kontakt von oben auf den Katalysator gepumpt, wo es über den Katalysator nach unten in einen Abscheider rieselte. Der Wasserstoff wurde im Gleichstrom von oben nach unten durch den Reaktor geleitet, wobei eine Teilmenge aus der Gasphase nach Verlassen des Reaktors entspannt wurde, um die Inertanteile aus dem System auszuschleusen. Ein Anteil der hydrierten Flüssigphase wurde als Rückführung wieder zum Kopf des Reaktors gepumpt. Die Reaktoraustrittstemperatur betrug 110°C. Nach 600 Betriebsstunden war noch die ursprüngliche Katalysatoraktivität vorhanden. Im Reaktionsprodukt war kein Nitrotoluol mehr nachweisbar. Kernhydriertes Amin war zu ca. 500 ppm enthalten. Der Gehalt an höhersiedenden Nebenprodukten betrug 350 ppm. Die Ausbeute an o-Toluidin betrug 99,7%.

### Beispiel 72

### Technische Hydrierung von Phenol

Durch einen mit hochgespanntem Dampf auf durchschnittlich 140°C erhitzten Röhrenreaktor (Rohrlänge 6 m; Rohrdurchmesser 50 mm; Anzahl der Rohre 366), der mit 4200 Litern eines nach Beispiel 18 hergestellten Katalysators mit 9 g Pd/Liter $\alpha$-Al$_2$O$_3$-Träger gefüllt war, wurde — nach vorhergegangener Aktivierung mit Wasserstoff (im allgemeinen 200°C, 10 h) — von unten nach oben im Wasserstoffstrom verdampftes Phenol geleitet (3000 kg Phenol/h; Molverhältnis Phenol zu Wasserstoff = 1 : 6). Dabei lagen die Kontakttemperaturen zwischen 140 und 190°C. Nach Passieren des Reaktors wurden die gasförmigen Reaktionsprodukte kondensiert. Aus dem im Kreislauf geführten Wasserstoff wurde eine Teilmenge zur Entfernung der Inertgasanteile ausgeschleust.

Das erhaltene Reaktionsprodukt bestand aus 96,2% Cyclohexanon und 3,7% Cyclohexanol. Der Phenolgehalt lag unter 0,05%. Bezogen auf 1 Liter Katalysator wurden 5300 kg Cyclohexanon erzeugt.

### Patentansprüche

1. Verfahren zur Herstellung von Trägerkatalysatoren durch Behandlung von inertem Trägermaterial mit einer Base, anschließendes Trocknen, Aufbringen einer Metallsalzlösung und gegebenenfalls Reduktion zum Metall, dadurch gekennzeichnet, daß man inertes Trägermaterial mit einer BET-Oberfläche von kleiner als 20 m²/g mit einer Base entsprechend der Saugfähigkeit des Trägermaterials bis zur Sättigung tränkt, anschließend bis zur Gewichtskonstanz trocknet, in an sich bekannter Weise mit einer Metallsalzlösung ebenfalls bis zur Sättigung tränkt, anschließend gegebenenfalls zum Metall reduziert, wobei man die Base in einer solchen Menge aufbringt, daß 0,01 bis 50 Grammäquivalente Base pro Grammäquivalent Metall vor der Tränkung mit der Metallsalzlösung im Träger enthalten sind und den Katalysator mit Wasser wäscht bis keine Ionen der bei der Katalysatorherstellung verwendeten Verbindungen im Waschwasser mehr nachweisbar sind.

2. Verfahren nach Anspruch 1, dadurch gekennzeichnet, daß man als inertes Trägermaterial Metalloxide, Silikate, Spineile, Carbide oder Carbonate sowie deren Mischungen einsetzt.

3. Verfahren nach Anspruch 1 und 2, dadurch gekennzeichnet, daß man die Trocknung bei Temperaturen von 50° bis 200°C vornimmt.

4. Verfahren nach Anspruch 1 bis 3, dadurch gekennzeichnet, daß man als Basen Oxide, Carbonate, Hydrogencarbonate, Hydrogenphosphate, Hydroxide, Alkoxide, Formiate oder deren Mischungen einsetzt.

5. Verfahren nach Anspruch 1 bis 4, dadurch gekennzeichnet, daß man mit der Salzlösung eines Metalles der Gruppen IIIa, IVa, Va, VIa, VIIa, VIIIa, Ib, IIb, IIIb, IVb oder Vb des Periodensystems bis zur Sättigung tränkt.

6. Verwendung der gemäß Anspruch 1 hergestellten Trägerkatalysatoren für Hydrierungen, Dehydrierungen, Hydrogenolysen, Oxidationen, Polymerisationen, Isomerisierungen oder Cyclisierungen.

7. Verwendung der gemäß Anspruch 1 hergestellten Trägerkatalysatoren für die katalytische Hydrierung von Nitrobenzol, Nitrotoluol, Dinitrobenzolen, Dinitrotoluolen, Trinitroluolen oder Nitrophenolen zu den entsprechenden aromatischen Aminen.

Claims

1. Process for the preparation of supported catalysts by the treatment of inert support material with a base, subsequent drying, application of a metal salt solution and, optionally reduction of the salt to the metal, characterised in that inert support material with a BET surface area of less than 20 m²/g is impregnated, in accordance with the absorbency of the support material, to saturation with a base, then dried to constant weight, impregnated in a manner known per se with a metal salt solution, also to saturation, and the metal salt is then optionally reduced to the metal, the base being applied in such a quantity that 0.01 to 50 gram equivalents of base per gram equivalent of metal are contained in the support before the impregnation with the metal salt solution and the catalyst is washed with water until no more ions of the compounds used in the preparation of the catalyst are detectable in the wash water.

2. Process according to Claim 1, characterised in that metal oxides, silicates, spinels, carbides or carbonates or also mixtures thereof are employed as the inert support material.

3. Process according to Claim 1 and 2, characterised in that drying is carried out at temperatures of 50° to 200°C.

4. Process according to Claim 1 to 3, characterised in that the bases employed are oxides, carbonates, bicarbonates, hydrogen phosphates, hydroxides, alkoxides or formates or mixtures thereof.

5. Process according to Claim 1 to 4, characterised in that impregnation to saturation is carried out with a salt solution of a metal of groups IIIa, IVa, Va, VIa, VIIa, VIIIa, Ib, IIb, IIIb, IVb or Vb of the periodic table.

6. Use of the supported catalysts prepared according to Claim 1 for hydrogenation, dehydrogenation, hydrogenolysis, oxidation, polymerisation, isomerisation or cyclisation reactions.

7. Use of the supported catalysts prepared according to Claim 1 for the catalytic hydrogenation of nitrobenzene, nitrotoluene, dinitrobenzenes, dinitrotoluenes, trinitrotoluenes or nitrophenols to the corresponding aromatic amines.

**Revendications**

1. Procédé pour la préparation de catalyseurs sur support par traitement d'un matériau de support inerte par une base, puis séchage, dépôt d'une solution de sel métallique et éventuellement réduction à l'état de métal, caractérisé en ce que l'on imprègne un matériau de support inerte ayant une surface spécifique BET de moins de 20 m²/g avec une base jusqu'à la saturation correspondant à la capacité d'absorption du metériau de support, ensuite on sèche jusqu'à poids constant, on imprègne de manière connue, également jusqu'à saturation, avec une solution del sel métallique et ensuite on réduit éventuellement à l'état de métal, la base étant déposée en quantité telle que le support contienne 0,01 à 50 équivalents-g de base par équivalent-g de métal avant l'imprégnation avec la solution de sel métallique et on lave le catalyseur par l'eau jusqu'à ce que l'on ne puisse plus déceler dans l'eau de lavage d'ions des composés utilisés dans la fabrication du catalyseur.

2. Procédé selon la revendication 1, caractérisé en ce que l'on utilise comme matériaux de support des oxydes métalliques, des silicates, des spinelles, des carbures ou des carbonates, ainsi que leurs mélanges.

3. Procédé selon la revendication 1 et 2, caractérisé en ce que l'on effectue le séchage à des températures de 50 à 200°C.

4. Procédé selon les revendications 1 à 3, caractérisé en ce que l'on utilise comme bases des oxydes, des carbonates, des bicarbonates, des hydrogènophosphates, des hydroxydes, des alcoolates, des formiates ou leurs mélanges.

5. Procédé selon les revendications 1 à 4, caractérisé en ce que l'on imprègne jusqu'à saturation avec la solution de sel d'un métal des groupes IIIa, IVa, Va, VIa, VIIa, VIIIa, Ib, IIb, IIIb, IVb ou Vb de la Classification Périodique des Eléments.

6. Utilisation des catalyseurs sur support préparés selon la revendication 1 pour des hydrogénations, des déshydrogénations, des hydrogénolyses, des oxydations, des polymérisations, des isomérisations ou des cyclisations.

7. Utilisation des catalyseurs sur support préparés selon la revendication 1 pour l'hydrogénation catalytique de nitrobenzène, de nitrotoluène, de dinitrobenzènes, de dinitrotoluènes, de trinitrotoluènes, ou de nitrophénols en les amines aromatiques correspondantes.